(19) Europäisches Patentamt
European Patent Office
Office européen des brevets

(11) Numéro de publication: **0 179 004 B1**

(12) # FASCICULE DE BREVET EUROPEEN

(45) Date de publication du fascicule du brevet: **13.07.88**

(51) Int. Cl.⁴: **C 07 F 15/00**

(21) Numéro de dépôt: **85420169.6**

(22) Date de dépôt: **24.09.85**

(54) **Complexes rhodiés, dinucléaires et hydrosolubles et leur utilisation comme catalyseur d'hydroformylation.**

(30) Priorité: **16.10.84 FR 8416007**

(43) Date de publication de la demande: **23.04.86 Bulletin 86/17**

(45) Mention de la délivrance du brevet: **13.07.88 Bulletin 88/28**

(84) Etats contractants désignés: **AT BE CH DE FR GB IT LI LU NL SE**

(56) Documents cités: **FR - A - 2 408 388** **FR - A - 2 478 078**

(73) Titulaire: **RHONE-POULENC CHIMIE, 25, quai Paul Doumer, F-92408 Courbevoie Cédex (FR)**

(72) Inventeur: **Besson, Bernard, 1 rue du Tonkin, F-69100 Villeurbanne (FR)**
Inventeur: **Kalck, Phillipe, "La Pradine" Tolozane No. 4 Auzeville, F-31320 Castanet Tolosan (FR)**
Inventeur: **Thorez, Alain, "Le Castagné" Baziege, F-31450 Montgiscard (FR)**

(74) Mandataire: **Varnière-Grange, Monique et al, RHONE-POULENC INTERSERVICES Service Brevets Chimie Centre de Recherches de Saint-Fons B.P. 62, F-69192 Saint-Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

EP 0 179 004 B1

**Description**

La présente invention a pour object des complexes rhodiés, dinucléaires et hydrosolubles, et l'utilisation de ces complexes ou de leurs solutions aqueuses comme catalyseur d'hydroformylation.

Des complexes rhodiés dinucléaires du type (1):

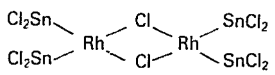 (1)

et leur utilisation comme catalyseur d'hydroformylation sont décrits dans le brevet US 3 501 581. Néanmoins ces catalyseurs sont peu actifs.

Des complexes rhodiés dinucléaires du type (2):

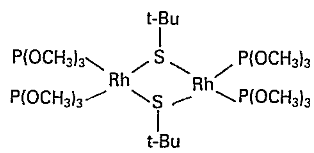 (2)

(dans lequel t-Bu représente un radical tertiobutyle) ou du type (3)

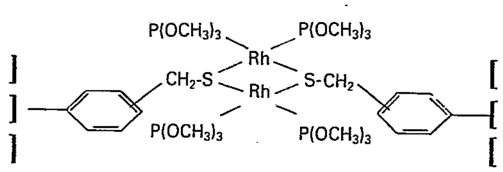 (3)

dans lequel:

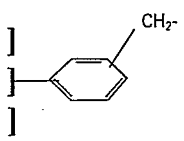

représente un reste provenant du polystyrène chlorométhylé:

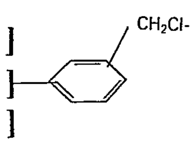

ainsi que leur utilisation comme catalyseur d'hydroformylation sont décrits dans le brevet US 4 215 066.

Toutefois si l'intérêt de principe de ces complexes rhodiés ne peut être mis en doute, le développement à l'échelle industrielle de leur untilisation comme catalyseurs est compromis par leur manque de sélectivité. En effet le rapport n/n + iso (n: aldéhyde normal linéaire; iso: aldéhyde branché) que permettent d'obtenir ces types de complexes en hydroformylation est notoirement insuffisant ce qui grève lourdement l'économie globale de ce procédé.

Par ailleurs la récupération et le recyclage des complexes du type (2) se heurtent aux difficultés classiquement associées aux réactions de catalyse homogène, difficultés rendues encore plus ardues par le fait qu'il est économiquement impensable de perdre la moindre quantité de rhodium.

On aurait pu penser palier ces inconvénients au moins en ce qui concerne la récupération et le recyclage du catalyseur par le recours à des complexes du type (3) ci-avant c'est-à-dire à des composés dans lesquels les complexes organo-métalliques sont «immobilisés» sur un support solide organique (ou minéral) par l'intermédiaire de groupes fonctionnels dudit support. Toutefois on observe généralement qu'une partie non négligeable du métal passe en solution dans la phase réactionnelle fluide (cf. notamment W. H. Lang et coll. J. Organomet. Chem., 134, 85, 1977).

Par ailleurs comme cela est enseigné par J. Falbe dans «New Synthesis with Carbon Monoxyde», Springer Verlag (1980), les masses catalytiques ainsi obtenues sont beaucoup plus sensibles aux phénomènes de désactivation et d'empoisonnement que les catalyseurs analogues solubles.

On voit donc que l'intérêt de ces «complexes supportés» ne peut être établi sur le plan industriel, d'autant plus que leur mise en œuvre implique des difficultés au niveau des transferts de matière et/ou de chaleur.

Il a maintenant été trouvé de nouveaux complexes rhodiés, dinucléaires et hydrosolubles; l'utilisation des dits complexes et/ou de leurs solutions aqueuses pour catalyser les réactions d'hydroformylation des oléfines permet d'obvier aux inconvénients des procédés antérieurs.

Ces nouveaux complexes rhodiés qui vont maintenant être décrits non seulement permettent d'atteindre des rapports n/n + iso élevés lors de leur utilisation comme catalyseurs d'hydroformylation avec une activité légèrement supérieure à celle observée, toutes conditions égales par ailleurs, lors de la mise en œuvre d'un complexe mononucléaire, mais encore autorisent une séparation aisée des différents termes de la réaction.

En effet, le fait que les réactifs et les produits de la réaction soient situés dans une phase fluide organique et/ou dans une phase gazeuse et que le système catalytique puisse se trouver intégralement (ou presque) dans une phase fluide aqueuse rend une séparation possible simplement en cessant d'agiter la masse réactionnelle et, le cas échéant, en relâchant la pression; le recours uniquement à des phases fluides favorise les transferts de matières et de chaleur.

La présente invention a donc pour objet des complexes rhodiés, dinucléaires et hydrosolubles de formule générale (I):

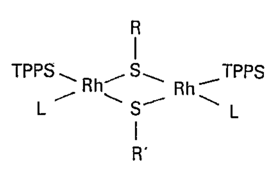 (I)

dans laquelle:

– R et R' identiques ou différents, représentent chacun un radical alkyle, aryle, arylalkyle ou alkylaryle renfermant au maximum 12 atomes de carbone, pouvant comporter un ou plusieurs substituants choisis parmi les atomes d'halogènes, les groupements sulfonate, carboxylate, cyano, amino disubstitué de formule $-NR_1R_2$ dans laquelle $R_1$ et $R_2$, identiques ou différents, représentent chacun un radical alkyle linéaire ou ramifié ayant au maximum 4 atomes de carbone, ammonium et phosphonium, et les radicaux alcoxy qui comportent de 1 à 4 atomes de carbone, R et R' pouvant par ailleurs former ensemble un radical unique divalent alkylène, alcénylène ou alkadiènylène, linéaire ou ramifié contenant de 2 à 6 atomes de carbone,

– TPPS est un coordinat de triphénylphosphine sulfonée et,

– L représente un coordinat carbonyle (CO) ou un coordinat TPPS, et l'utilisation de ces complexes ou de leurs solutions aqueuses pour catalyser les réactions d'hydroformylation des composés insaturés.

Les coordinats de triphénylphosphines sulfonées plus particulièrement appropriés peuvent être représentés par la formule (II)

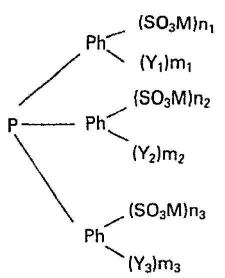
(II)

dans laquelle:

– Ph représente un groupement phényle,

– $Y_1$, $Y_2$, $Y_3$, identiques ou différents, représentent chacun un radical alkyle, linéaire ou ramifié ayant de 1 à 4 atomes de carbone, un radical alcoxy comportant de 1 à 4 atomes de carbone, un atome d'halogène, un groupe hydroxyle, un groupe nitrile, un groupe nitro ou un groupe amino disubstitué de formule $-NR_1R_2$ dans laquelle $R_1$ et $R_2$, ont la signification précédemment indiquée,

– M est un reste cationique d'origine minérale ou organique, choisi dans le groupe constitué par l'hydrogène et les cations minéraux dérivés de métaux alcalins ou alcalino-terreux, ou dérivés du plomb, du zinc ou du cuivre, le cation ammonium ($NH_4^+$), les cations ammonium quaternaire,

– $m_1$, $m_2$, et $m_3$ sont des nombres entiers identiques ou différents, pouvant être nuls, inférieurs ou égaux à 5.

– $n_1$, $n_2$, et $n_3$ sont des nombres entiers identiques ou différents, pouvant être nuls, inférieurs ou égaux à 3, l'un au moins des nombres $n_1$ à $n_3$ étant supérieur ou égal à 1.

Ces coordinats sont des produits connus plus particulièrement décrits dans le brevet français n° 2 314 910 et son addition n° 2 349 562.

Ces deux documents mettent également en évidence l'intérêt de ces composés dans le cadre des réactions d'hydroformylation catalysée par une source de rhodium et de celui du recyclage du catalyseur par l'intermédiaire d'un système biphasique comportant une phase aqueuse renfermant le catalyseur et le ou les composés en cause.

Les coordinats TPPS préférés répondent à la formule (II) ci-avant dans laquelle:

– $Y_1$, $Y_2$ et $Y_3$, identiques ou différents représentent des groupements choisis parmi les radicaux alkyles ayant de 1 à 2 atomes de carbone, les radicaux alcoxy ayant de 1 à 2 atomes de carbone et le chlore,

– M représente un cation choisi dans le groupe comprenant le cation hydrogène, le cation hydrogène, le cation ammonium, les cations dérivés du sodium, du potassium, du calcium et du baryum et les cations ammonium quaternaire de formule $N(R_3R_4R_5R_6)$ dans laquelle $R_3$, $R_4$, $R_5$ et $R_6$, identiques ou différents représentent des radicaux alkyles, linéaires ou ramifiés comportant au maximum 4 atomes de carbones,

– $m_1$, $m_3$ et $m_3$ sont des nombres entiers compris entre 0 et 3, inclus.

Les coordinats TPPS plus particulièrement préférés répondent à la formule (II) ci-avant dans laquelle:

– $m_1$, $m_2$ et $m_3$ sont nuls,

– M est choisi parmi les cations dérivant du sodium, du potassium, du calcium, du baryum, le cation ammonium et les cations tétraméthyl- et trétraéthylammonium;

– le(s) groupement (s) $SO_3$ est (sont) en position méta.

Comme autres exemples de coordinats TPPS appropriés on peut citer: les sels alcalins ou alcalino-terreux, les sels d'ammonium, les sels d'ammonium quaternaires des phosphines sulfonées suivantes:

(m-sulfophényl)diphénylphosphine; (p-sulfophényl)diphénylphosphine;

(m-sulfo, p-méthylphényl)di(p-méthylphényl)-phosphine;

(m-sulfo, p-méthoxyphényl)di(p-méthoxyphényl)-phosphine;

(m-sulfo, p-chlorophényl)di(p-chlorophényl)phosphine;

di(m-sulfophényl)phénylphosphine;

di(p-sulfophényl)phénylphosphine;

di(m-sulfo, p-méthylphényl) (p-méthylphényl)-phoshpine;

di(m-sulfo, p-méthoxyphényl) (p-méthoxyphényl)phosphine;

di(m-sulfo, p-chlorophényl) (p-chlorophényl)-phosphine;

tri(m-sulfophényl)phosphine;

tri(p-sulfophényl)phosphine;

tri(m-sulfo, p-méthylphényl)phosphine;

tri(m-sulfo, p-méthoxyphényl)phosphine;

tri(m-sulfo, p-chlorophényl)phosphine;

(o-sulfo, p-méthylphényl) (m-sulfo, p-méthylphényl) (m, m'-disulfo, p-méthylphényl)phosphine;

(m-sulfophényl) (m-sulfo, p-chlorophényl) (m, m'-disulfo, p-chlorophényl)phosphine;

Bien entendu, on peut recourir à des mélanges de ces phosphines. En particulier on peut utiliser un mélange de (m-sulfophényl)diphénylphosphine, de di(m-sulfophényl)phénylphosphine et de tri(m-sulfophényl)phosphine.

Les complexes rhodiés dinucléaires et hydrosolubles, selon la présente invention, comportent deux ponts mu-thiolato représentés par -SR et -SR' dans la formule générale (I); R et R', identiques ou différents, représentent chacun un radical alkyle, aryle, arylalkyle ou alkylaryle renfermant au maximum 12 atomes de carbone, pouvant comporter un ou plusieurs substituants choisis parmi les atomes d'halogènes, les groupements sulfonate, carboxylate, cyano, amino disubstitué de formule $-NR_1R_2$ dans laquelle $R_1$ et $R_2$ ont la signification précédemment donnée, ammonium et phosphonium, et les radicaux alcoxy qui comportent de 1 à 4 atomes de carbone, R et R' pouvant par ailleurs former ensemble un radical unique divalent alkylène, alcénylène ou alkadiénylène, linéaire ou ramifié contenant de 3 à 6 atomes de carbone.

R et R' sont de préférence identiques. Ils sont en outre avangateusement choisis parmi les radicaux alkyles comportant au maximum 4 atomes de carbone et le radical benzyle; R et R' sont avantageusement chacun un radical tertiobutyle.

Les complexes rhodiés dinucléaires et hydrosolubles, selon la présente invention comportent également un coordinat L par atome de rhodium. L est avantageusement un coordinat carbonyle.

Les complexes en cause peuvent être préparés par des méthodes connues en elles-mêmes à partir de complexes dinucléaires du rhodium à pont mu-chloro tel que le di-mu-chlorotétracarbonyldirhodium (I), lui même préparé comme indiqué dans Inorganic Syntheses Vol 8 (1968) page 211.

Pour ce faire le di-mu-chlorotétracarbonyldirhodium (I) est mis à réagir soit sur le(s) thiol(s) de formule (s) HSR (et HSR') soit sur un(des) thiolate(s) de lithium (par exemple) pour donner naissance au complexe de structure apparentée aux ponts mu-thiolato qui est alors mis à son tour à réagir sur le coordinat TPPS pour conduire finalement au complexe recherché.

Les réactions en cause peuvent être représentées comme suit:

$[Rh(mu\text{-}Cl)(CO)_2]_2 + 2\,HRS \longrightarrow [Rh(mu\text{-}SR)(CO)_2]_2 + 2\,HCl$ (1)

$[Rh(mu\text{-}Cl)(CO)_2]_2 + 2\,LiSR \longrightarrow [Rh(mu\text{-}SR)(CO)_2]_2 + 2\,LiCl$ (2)

$[Rh(mu\text{-}SR)(CO)_2]_2 + 2\,TPPS \longrightarrow [Rh(mu\text{-}SR)(CO)(TPPS)]_2 + 2\,CO$ (3)

dans lesquelles R et TPPS ont les significations précédemment indiquées. Il est également possible de partir du di-mu-chlorobis(cyclooctadiène-1,5) dirhodium (I) $[Rh\ Cl\ (COD)]_2$ pour former de manière analogue le complexe $[Rh(mu\text{-}SR)(COD)]_2$ qui alors mis à réagir sur le coordinat TPPS selon la réaction (4) ci-après:

$[Rh(mu\text{-}SR)(COD)]_2 + 4\,TPPS \longrightarrow [Rh(mu\text{-}SR)(TPPS)_2]_2 + 2\,COD$ (4)

Bien entendu, si R et R' sont distincts on fera appel aux deux thiols (ou thiolates) correspondants.

Il est a noter que si les thiols utilisés dans la réaction (1) sont des produits du commerce, les coordinats TPPS sont des produits connus qui peuvent être préparés selon les procédés également connus. Ainsi, conformément à l'enseignement de H. Schindlbauer, Monatsch. Chem. 96, pages 2051–2057 (1965), on peut préparer le sel de sodium de la (p-sulfophényl)diphénylphosphine en faisant réagir, en présence de sodium ou de potassium, du p-chlorobenzène sulfonate de sodium avec la diphénylchlorophosphine. Selon la méthode décrite dans J. Chem. Soc. pages 276–288 (1958) et dans le brevet anglais n° 1 066 261, on peut préparer des phénylphosphines de formule (II) en faisant appel à la réaction de sulfonation de noyaux aromatiques à l'aide d'oléum, puis en procédant à la neutralisation des groupes sulfoniques formés au moyen d'un dérivé basique approprié d'un des métaux que représente M dans la formule (II). La phosphine sulfonée brute obtenue peut contenir en mélange l'oxyde de phosphine sulfonée correspondant, dont la présence n'est cependant pas génante pour l'utilisation envisagée, quoique non souhaitable.

Dans l'hypothèse où l'on désire utiliser de la tri (m-sulfophényl)phosphine de qualité améliorée, c'est-à-dire renfermant le moins possible d'oxydes [degré V d'oxydation du phosphore ($P^V$)] on aura tout avantage à la préparer selon la technique décrite dans le brevet europeen n° 0 104 967.

Dans l'un de ses aspects, la présente invention concerne l'utilisation des complexes rhodiés dinucléaires et hydrosolubles de formule (I) comme catalyseurs ou précuseurs des espèces métalliques catalytiquement actives dans un procédé d'hydroformylation de composés organiques comportant au moins une double liaison carbone-carbone.

Dans un autre de ses aspects la présente invention concerne l'utilisation des complexe rhodiés en cause comme catalyseurs ou précuseurs des espèces métalliques catalytiquement actives dans un procédé d'hydroformylation de composés organiques comportant au moins une double liaison carbone-carbone, sous forme de leurs solutions aqueuses.

Le procédé d'hydroformylation des composés organiques qui comportent au moins une double liaison carbone-carbone est conduit en phase liquide en vue de former des composés aldéhydiques et comprend la mise en contact d'au moins un composé à double liaison carbone-carbone avec de l'oxyde de carbone et de l'hydrogène en présence d'une solution aqueuse d'un complexe rhodié, binucléaire de formule (I) ci-avant et le cas échéant d'une phosphine sulfonée de formule (II) ci-avant.

Les composés organiques comportant au moins une double liaison carbone-carbone susceptibles d'être hydroformylés comprennent les composés monoéthyléniques aliphatiques ayant de 2 à 20

atomes de carbone, incluant des oléfines à double liaison terminale ou interne, linéaires ou ramifiées, les diènes conjugués présentant dans leur molécule le squelette du butadiène -1,3 et le styrène.

A titre d'exemples, on peut citer:
parmi les hydrocarbures éthyléniques, l'éthylène, le propylène, le butène-1, le méthyl-2 butène-1, le butène-2, le pentène-1, le pentène-2, l'hexène-1, l'éthyl-3 hexène-1, le propyl-2 hexène-1, l'hexène-2, l'heptène-1, l'octène-1, l'octène-3, le diméthyl-4,4 nonène-1, le décène-1, le décène-2, le propyl-6 décène-1, l'undécène-3, le dodécène-1, le tétradécène-5, l'octadécène-1, l'octadécène-2;
parmi les diènes conjugués présentant dans leur molécules le squelette butadiène-1,3: le butadiène-1,3, l'isoprène, le pipérylène, l'hexadiène-1,3, l'hexadiène-2,4, le chloroprène, le cyclohexyl-1 butadiène-1,3, le phényl-1 butadiène-1,3, l'octadiène-2,4, le méthyl-3 pentadiène-1,3, le méthyl-2 pentadiène-2,4, le cyclohexadiène-1,3, le cyclooctadiène-1,3, éventuellement substitués par le groupe alcoxycarbonyle, tel que le pentadiène-2,4, oate de méthyle.

Le procédé d'hydroformylation selon la présente invention s'applique tout particulièrement aux composés mono-éthyléniques aliphatiques linéaires, comportant de 2 à 8 atomes de carbone, comme l'éthylène, le propylène, le butène-1, le pentène-1, l'hexène-1, l'hexène-2, l'octène-1.

La quantité de complexe rhodié est en général comprise entre 0,0001 et 0,25 et de préférence 0,0005 et 0,05 mole par litre de solution réactionnelle.

Selon une variante avantageuse, la solution aqueuse renferme en plus du complexe rhodié binucléaire une phosphine sulfonée.

Pour ce qui concerne la phosphine sulfonée dont une solution aqueuse est utilisée pour la mise en œuvre du procédé d'hydroformylation on se reportera à ce qui vient d'être exposé à propos des triarylphosphines sulfonées utilisées comme coordinat des complexes rhodiés, dinucléaires et hydrosolubles de formule (I).

En effet, si la nature précise des phosphines sulfonées peut différer selon qu'elles sont utilisées comme coordinat des complexes en cause ou sous forme de leurs solutions aqueuses pour la mise en œuvre du procédé d'hydroformylation, la définition générale en reste la même, et de manière avantageuse on utilisera la même phosphine sulfonée ou le même mélange de phosphines sulfonées pour préparer ex-temporanément les complexes rhodiés de formule (I) et pour mettre en œuvre le procédé d'hydroformylation. Dans ce dernier cas on pourra même envisager la synthèse in situ du complexe de formule (I) dans les conditions d'hydroformylation à partir de la phosphine sulfonée en cause et d'un composé $[Rh(mu\text{-}SR)(CO)_2]_2$ (précurseur) dont il a déjà été question précédemment (cf. réaction 3), voire même de former in situ ce précurseur à partir du complexe de structure apparentée aux ponts muchloro et d'un thiol (ou thiolate) approprié (cf. les réactions 1 et 2).

La quantité de phosphine sulfonée de formule (II) utilisée pour préparer la solution réactionnelle est choisie de telle sorte que le rapport atomique du phosphore au degré d'oxydation III au rhodium $P^{III}/Rh$ soit compris entre 1 et 300 et de préférence entre 1,5 et 100.

Bien que la réaction soit de préférence conduite dans l'eau, il peut être avantageux d'employer un solvant organique inerte et notamment un solvant miscible à l'eau, ayant de préférence un point d'ébullition inférieur à celui de l'eau, utilisé en quantité telle qu'il permet d'accroître le cas échéant la solubilité de l'oléfine dans la solution aqueuse catalytique, sans toutefois rendre miscibles dans la phase aqueuse les produits aldéhydiques formés. Comme solvants utilisables figurent des composés monohydroxylés aliphatiques saturés, linéaires ou ramifiés tels que l'alcool méthylique, l'alcool éthylique, l'alcool propylique, l'alcool isopropylique; des cétones aliphatiques saturées telle que l'acétone, des nitriles aliphatiques inférieurs comme l'acétonitrile ainsi que l'éther méthylique du diéthylèneglycol et le diméthoxyéthane.

On peut également utiliser un solvant non miscible à l'eau comme le benzène, le toluène, le benzonitrile, l'acétophénole, l'éther éthylique, l'éther propylique, l'éther isopropylique, l'octane, la méthyléthylcétone et le propionitrile.

La température à laquelle est conduite la réaction peut varier dans de larges limites. Plus particulièrement, on opère à des températures modérément élevées qui peuvent varier entre 20 °C et 150 °C, et de préférence entre 50 °C et 120 °C.

La valeur de la pression totale d'hydrogène et d'oxyde de carbone qui est requise pour la mise en œuvre du procédé peut être celle de la pression atmosphérique bien que des pressions plus élevées soient préférées; des pressions totales comprises entre 1 et 200 bars et de préférence entre 10 et 100 bars conviennent bien en général.

Les pressions partielles d'oxyde de carbone et d'hydrogène dans le mélange de gaz utilisée sont telles que la rapport molaire oxyde de carbone/hydrogène varie entre 0,1 et 10; de préférence, on utilise un rapport molaire qui varie entre 0,2 et 5.

Une manière pratique de mettre en œuvre le procédé de l'invention consiste à charger dans un réacteur résistant à la pression au préalable purgé à l'aide d'un gaz inerte (azote ou argon) soit la solution aqueuse catalytique préalablement formée soit les divers composants: un complexe rhodié binucléaire et hydrosoluble de formule (I) ou un précurseur défini ci-avant, l'eau, le cas échéant, la phosphine sulfonée en solution aqueuse et, si cela s'avère souhaitable le solvant organique. On charge alors le composé organique comportant ou moins une double liaison carbone-carbone. On porte alors le réacteur à la température de réaction avant ou après l'introduction de l'oxyde de carbone et de l'hydrogène qui eux mêmes peuvent être introduits simultanément ou séparément, avant ou après, voire en même temps que le composé insaturé.

Après arrêt de la réaction, on refroidit à la température ambiante voisine de 20°C et on détend l'excès de gaz qu'il contient. Le contenu du réacteur est alors retiré et il suffit ensuite d'isoler les produits aldéhydiques en procédant, à une décantation et éventuellement à un lavage à l'aide d'un solvant convenable tel que par exemple l'éther diéthylique, le benzène, le toluène. On peut également séparer les produits aldéhydiques du mélange résiduel, au besoin après filtration, par extraction à l'aide d'un des solvants précités. Bien que la décantation et l'extraction soient des modes de traitement préférés, il est encore possible de faire appel à la technique de distillation pour isoler les produits aldéhydiques formés.

La solution aqueuse résiduelle peut être recyclée dans le réacteur pour catalyser une nouvelle opération d'hydroformylation. Le procédé selon l'invention convient particulièrement bien à la mise en œuvre continue.

Les exemples ci-après illustrent l'invention:

Exemples

Exemples de complexes selon l'invention

Exemple 1:

Cet exemple illustre la préparation d'un complexe selon l'invention comportant deux ponts mu-tertiobutylthiolato et deux coordinats de tri(m-sulfophényl)phosphine sous forme de son sel de sodium (TPPTS)

a) Synthèse de
$[Rh(mu\text{-}S\text{-}t\text{-}Bu)(Co)_2]_2$

A 0,390 g ($10^{-3}$mole) de di-mu-chlorotétracarbonyldirhodium (I) en solution dans 20 ml de toluène, on ajoute un léger excès (2,2 $10^{-3}$ mole) de tertiobutylthiolate de lithium (obtenu par action de n-butyllithium sur le t-butylthiol) dans 10 ml de toluène. La solution devient immédiatemment d'un jaune lumineux tandis qu'un précipité blanc ténu apparaît. Après 15 minutes de réaction, le toluène est distillé sous pression réduite et le composé est extrait 3 fois par 10 ml d'hexane et est séparé par filtration du chlorure de lithium.

Le rendement est quantitatif.

b) Synthèse du complexe
$[Rh(mu\text{-}S\text{-}t\text{-}Bu)(CO)(TPPTS)]_2$

A une solution de 0,496 g ($10^{-3}$ mole) de di(mu-tertiobutyl-thiolato)tétracarbonyldirhodium (I), obtenu en a), dans 30 ml de méthanol on ajoute 1,23 g de TPPTS titrant 1,63.$10^{-3}$ équivalent de phosphore au degré d'oxydation 3 par gramme (soit 2.$10^{-3}$ équivalent de $P^{III}$) renfermant de la di(m-sulfophényl) phénylphosphine sous forme de son sel sodique [le rapport molaire de la tri(m-sulfophényl)phosphine à la di(m-sulfophényl)phénylphosphine étant de 62/27].

Un dégagement gazeux se produit et la solution devient jaune plus soutenu. On laisse sous agitation jusqu'à consommation totale de la phosphine, puis on distille le solvant sous pression réduite. Le composé obtenu est jaune vif. Le rendement est quantitatif.

L'examen par spectroscopie infra-rouge d'une solution dudit composé dans le méthanol montre l'existence d'une vibration d'élongation du groupement carbonyle, une bande de très forte intensité à 1967 $cm^{-1}$ et une bande de forte intensité à 1955 $cm^{-1}$ étant observées. L'analyse par résonance magnétique nucléaire du $p^{31}$ montre l'existence d'un doublet à 36,5 ppm avec une constante de couplage Rh-P de 152 Hz.

Ce complexe sera désigné dans ce qui suit par «complexe 1».

Exemple 2:

Cet exemple illustre la préparation d'un complexe selon l'invention comportant 2 ponts mu-phénylthiolato et deux coordinats de TPPTS.

a) Synthèse de
$[Rh(mu\text{-}S\text{-}Ph.)(CO)_2]_2$ (Ph. = phényle):

A 0,390 g ($10^{-3}$ mole) de di-mu-chlorotétracarbonyldirhodium (I) dans 20 ml d'hexane on ajoute lentement sous contre-courant d'azote 0,206 ml (2.$10^{-3}$ mole) de thiophénol. La solution jaune se colore instantanément en rouge et des paillettes rouge-vif précipitent peu à peu. Après 30 minutes de réaction on élimine par filtration la solution jaune et on sèche sous vide le composé obtenu.

Le rendement est quantitatif.

b) Synthèse du complexe
$[Rh(mu\text{-}S\text{-}Ph.)(CO)(TPPTS)]_2$ (Ph. = phènyle):

A 0,536 g ($10^{-3}$ mole) de de-(mu-phénylthiolato)-tétracarbonyl-dirhodium (I) obtenu en a), mis en suspension dans 20 ml de méthanol on ajoute 1,23 g TPPTS dont les caractéristiques ont été indiquées précédemment, en solution dans 10 ml d'eau. Peu à peu, la solution se colore en jaune brun tandis qu'un dégagement gazeux se produit. On laisse sous agitation jusqu'à dissolution complète puis, une fois la solution homogène, on distille le solvant sous pression réduite. Le rendement est quantitatif.

L'examen par spectroscopie infra-rouge d'une dispersion dudit complexe dans le bromure de césium montre la présence d'une bande large de très forte intensité à 1980 $cm^{-1}$.

Ce complexe sera désigné dans ce qui suit par «complexe 2».

Exemple 3:

De manière analogue on prépare le complexe:
$[Rh(mu\text{-}S\text{-}CH_3)(CO)(TPPTS)]_2$

A partir du di(-mu-méthylthiolato)tétracarbonyl-dirhodium (5) et de TPPTS dont les caractéristiques ont été indiquées à l'exemple 1 ci-avant.

L'examen par spectroscopie infra-rouge d'une dispersion dudit complexe dans le Nujol montre la présence d'une bande large, de très forte intensité à 1970 $cm^{-1}$.

Ce complexe sera désigné dans ce qui suit par «complexe 3».

Exemple 4:

De manière analogue on prépare le complexe:
$[Rh(mu\text{-}S\text{-}CH_2\text{-}Ph.)(CO)(TPPTS)]_2$

A partir de di(-mu-benzylthiolato)tétracarbonyldirhodium (I) et de TPPTS dont les caractéristiques ont été indiquées à l'exemple 1 ci-avant.

L'examen par spectroscopie infra-rouge d'une dispersion dudit complexe dans le bromure de potassium montre la présence d'une bande large et de très forte intensité, à 1962 $cm^{-1}$.

Ce complexe sera désigné dans ce qui suit par «complexe 4».

Exemple 5:
De manière analogue on prépare le complexe.
$[Rh(mu\text{-}S\text{-}C_6F_5)(CO)(TPPTS)]_2$

A partir du di[mu-(pentafluorophényl)thiolato]-tétracarbonyldirhodium (I) et de TPPTS dont les caractéristiques ont été indiquées à l'exemple 1 ci-avant.

Ce complexe sera désigné dans ce qui suit par «complexe 5».

Exemple 6:
De manière analogue on prépare le complexe de formule:

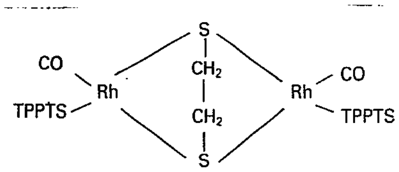

en deux stades à partir du di(mu-chloro)tétracarbonylrhodium (I) en le faisant réagir dans un premier stade sur l'éthanedithiol-1,2 pour former le complexe:

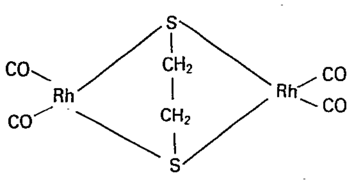

qui est alors mis à réagir, dans un deuxième stade sur la TPPTS. Le rendement est quantitatif.

L'examen par spectroscopie infra-rouge d'une dispersion dudit complexe dans le Nujol montre la présence d'une bande large et de très forte intensité, à 1996 $cm^{-1}$.

Ce complexe sera désigné dans ce que suit par «complexe 6».

Exemples d'utilisation de complexes selon l'invention dans les réactions d'hydroformylation:

Mode opératoire dans le cas de réactifs organiques gazeux dans les conditions normales de température et de pression (ex: propylène, butadiène...)

Dans de l'eau distillée et désaérée (20 ml) on ajoute de la tri(m-sulfophényl)phosphine sous forme de son sel de sodium et le composé du rhodium dans les quantités désirées. La solution catalytique est alors transférée par siphonnage sous argon dans un autoclave de 125 $cm^3$ en acier inoxydable. La quantité de réactif organique gazeux désirée est alors transférée dans l'autoclave au moyen d'un sas. L'autoclave est ensuite pressurisé par un mélange gazeux constitué de CO et de $H_2$ dans le rapport volumique voulu, puis fermé sur lui-même. La pression totale imposée à température ambiante est telle qu'après montée en température la pression totale obtenue soit légèrement inférieure à la pression totale désirée. L'agitation de la masse réactionnelle est effectuée par secousses. Lorsqu'on atteint la température voulue, on alimente l'autoclave à pression constante à partir d'une réserve contenant le mélange gazeux (CO et $H_2$ dans un rapport volumique de 1). Après un temps de réaction donné, l'autoclave est ramené à température ambiante et la pression est relachée lentement. La masse réactionnelle est alors transférée sous argon par siphonnage dans une ampoule à décanter. Elle se sépare en deux phases. La phase organique est analysée par chromatographie en phase gazeuse. La phase aqueuse renfermant le système catalytique peut eventuellement être recyclée.

Mode opératoire dans le cas de réactifs organiques liquides dans des conditions normales de température et de pression.

La seule différence réside dans le mode d'introduction du réactif. Celui-ci est introduit dans le réacteur de la même manière et en même temps que la phase aqueuse catalytique. (L'utilisation d'un sas est devenue inutile.)

Les conventions utilisées dans les exemples ci-après sont les suivantes:

Tl = désigne le taux de linéarité parmi les aldéhydes obtenus, défini comme étant le rapport n/(n + iso). Il est exprimé en pourcents.

Pr = désigne la productivité spatiale exprimée en gramme de n-aldéhyde obtenu par heure et par litre de solution catalytique aqueuse.

M(ph. Org) = désigne la masse de la phase organique obtenue après décantation, exprimée en gramme.

RR = nombre de moles d'aldéhyde linéaire formées rapporté au nombre de moles du substrat engagées.

ND = non déterminable

Exemples 7 à 24:

Une serie d'essais d'hydroformylation est réalisée sur le propylène avec un rapport $CO/H_2$ de 1/1 (en volume) à 120 °C, sous une pression en température de 50 bars environ, en présence de TPPTS dont les caractéristiques ont été indiquées à l'exemple 1, pour les exemples 7 à 20, la TPPTS des exemples 21 à 24 ayant des caractéristiques suivantes: elle se présente sous forme d'une solution aqueuse renfermant 0,59 mole/litre de $P^{III}$ à 97% (molaire) de tri(m-sulfophényl)phosphine sous forme de son sel de sodium et en présence de 0,097 $10^{-3}$ atome-gramme (At-g) de rhodium engagé sous forme de complexe dont la nature précise est indiquée dans le tableau I ci -après, chaque exemple pour lequel figure la mention «recycle» étant réalisé en recyclant la phase aqueuse catalytique récupérée par décantation en fin de l'essai qui le précède immédiatement.

Ainsi l'exemple 10 est réalisé en utilisant comme solution catalytique, la phase aqueuse récoltée en fin de l'essai décrit à l'exemple 9.

Les résultats obtenus ainsi que les conditions particulières sont rassemblés dans le tableau I ci-après.

Les exemples 7, 8, 11 et 12 réalisés avec le dimère du chlorocyclooctadiène-1,5 rhodium $[RhCl(COD)]_2$, qui ne font pas partie de la présente invention sont donnés à titre comparatif.

Tableau I

| EX No | Propylène $10^{-3}$Mol | Nature du catalyseur | P/Rh | Durée en h | M(Ph. org) | Pr | Tl |
|---|---|---|---|---|---|---|---|
| 7 | 190 | $[RhCl(COD)]_2$ | 100 | 2 | 1,6 | 36 | 95 |
| 8 | 190 | recyle | 100 | 2,3 | 2,5 | 48 | 96 |
| 9 | 202 | complexe 1 | 100 | 1,5 | 4,1 | 136 | 96 |
| 10 | 190 | recycle | 100 | 1,5 | 4,0 | 127 | 95 |
| 11 | 167 | $[RhCl(COD)]_2$ | 50 | 1 | 1,1 | 50 | 92 |
| 12 | 178 | recycle | 50 | 1 | 2,9 | 130 | 92 |
| 13 | 190 | complexe 1 | 50 | 1 | 5,5 | 278 | 96 |
| 14 | 190 | recycle | 50 | 1 | 11,1 | 384 | 93 |
| 15 | 190 | recycle | 50 | 1 | 8,5 | 364 | 92 |
| 16 | 179 | complexe 4 | 50 | 1 | 6,3 | 281 | 92 |
| 17 | 428 | recycle | 50 | 2,9 | 21,8 | 330 | 93 |
| 18 | 190 | recycle | 50 | 1 | 7,6 | 335 | 92 |
| 19 | 190 | complexe 2 | 50 | 1 | traces | ND | ND |
| 20 | 190 | recycle | 50 | 1 | 6,4 | 182 | 93 |
| 21 | 190 | complexe 3 | 50 | 1 | 7,1 | 303 | 93 |
| 22 | 179 | recycle | 50 | 1 | 11,7 | 522 | 93 |
| 23 | 428 | complexe 6 | 50 | 1 | 4,3 | 190 | 95 |
| 24 | 190 | complexe 5 | 50 | 1 | 6,0 | 180 | 93 |
| 25 | 288 | complexe 1 | 10 | 1 | 10,1 | 527 | 95 |
| 26 | 250 | recycle | 10 | 1 | 14,9 | 588 | 94 |

Exemples 27 à 31:

Une seconde série d'essais à été réalisée en utilisant le complexe 1 dont la préparation est décrite à l'exemple 1 dans des réactions d'hydroformylation conduites sur des substrats de nature diverse précisée dans le tableau II ci-après, avec un mélange $CO/H_2$ dans un rapport volumique de 1 en présence de TPPTS dont les caractéristiques ont été données à l'exemple 1, le rapport molaire P/Rh étant de 10, la pression mesurée à la température (indiquée par T dans le tableau II) étant égale à 50 bars. Les résultats obtenus ainsi que les conditions particulières figurent dans le tableau II ci-après.

Dans l'exemple 28 on obtient outre l'aldéhyde linéaire (nonanal) du méthyloctanal (39% molaire par rapport au produit engagé) et de l'éthylheptanal (24%).

Dans l'exemple 31 on obtient outre le phényl-3 propanal, du phényl-2 propanal (62%).

Exemples 32 à 35:

Une troisième série d'essais à été réalisée en utilisant ce même complexe 1 dans l'hydroformylation de l'hexène-1. La solution catalytique renfermant 0,194 $10^{-3}$ At-g de rhodium, 1,94 $10^{-3}$ mole de tri(m-sulfophényl)phosphine sous forme de son sel de sodium et dont les caractéristiques ont été indiquées à l'exemple 1, (P/Rh = 10) et 2 ml de méthanol, la température étant de 120 °C, la pression totale en température étant variable ainsi que le rapport volumique $H_2/CO$. Les résultats obtenus ainsi que les conditions particulières sont l'objet du tableau III ci-après.

Tableau II

| EX | Substrat | | Rh | T | Durée | M(Ph. Org) | RR |
|---|---|---|---|---|---|---|---|
| No | Nature | $10^{-3}$Mol | (*) | (°C) | en H | | |
| 27 | hexène-1 | 80 | 0,194 | 120 | 1 | 6,4 | 26,3 |
| 28 | octène-2 | 19 | 0,388 | 150 | 16 | 2,4 | 11 |
| 29 | butadiène-1,3 | 130 | 0,194 | 80 | 2 | 5,3 | 35,3 |
| 30 | isoprène | 50 | 0,194 | 120 | 2 | 1,8 | 23,4 |
| 31 | styrène | 44 | 0,194 | 120 | 1 | 5,1 | 15 |

(*): $10^{-3}$ At-g

Tableau III

| EX No | Pression (bar) | $H_2/CO$ (vol.) | Durée en H | M(Ph. Org) | RR | TI |
|---|---|---|---|---|---|---|
| 32 | 50 | 0,4 | 2 | 3,2 | 51 | 84 |
| 33 | 80 | 2 | 2,2 | 4,4 | 63 | 87 |
| 34 | 50 | 2,5 | 2 | 3,6 | 65 | 92 |
| 35 | 95 | 0,5 | 2 | 3,2 | 42 | 82 |

**Revendications pour les Etats contractants BE, CH, DE, FR, GB, IT, LI, LU, NL, SE**

1. Complexes rhodiés, dinucléaire et hydrosolubles de formule générale (I):

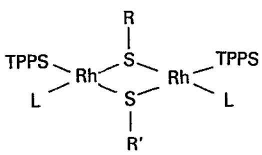

(I)

dans laquelle:
– R et R′ identiques ou différents, représentent chacun un radical alkyle, aryle, arylalkyle ou alkylaryle renfermant au maximum 12 atomes de carbone, pouvant comporter un ou plusieurs substituants choisis parmi les atomes d'halogènes, les groupements sulfonate, carboxylate, cyano, amino disubstitué de formule $-NR_1R_2$ dans laquelle $R_1$ et $R_2$, identiques ou différents, représentent chacun un radical alkyle linéaire ou ramifié ayant au maximum 4 atomes de carbone, ammonium et phosphonium, et les radicaux alcoxy qui comportent de 1 à 4 atomes de carbone, R et R′ pouvant par ailleurs former ensemble un radical unique divalent alkylène, alcénylène ou alkadiénylène, linéaire ou ramifié contenant de 2 à 6 atomes de carbone,
– TPPS est un coordinat de triphénylphosphine sulfonée et,
– L représente un coordinat carbonyle (CO) ou un coordinat TPPS.

2. Complexes de formule (I) selon la revendication 1, caractérisés en ce que le coordinat TPPS est au moins une triphénylphosphine sulfonée de formule générale (II):

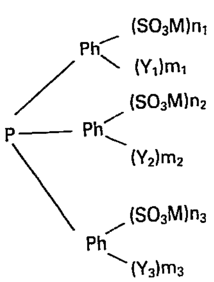

(II)

dans laquelle:
– Ph représente un groupement phényle
– $Y_1$, $Y_2$, $Y_3$, identiques ou différents, représentent chacun un radical alkyle, linéaire ou ramifié ayant 1 à 4 atomes de carbone, un radical alcoxy comportant de 1 à 4 atomes de carbone, un atome d'halogène, un groupe hydroxyle, un groupe nitrile, un groupe nitro ou un groupe amino disubstitué de formule $-NR_1R_2$ dans laquelle $R_1$ et $R_2$, identiques ou différents, représentent chacun un radical alkyle linéaire ou ramifié ayant au maximum 4 atomes de carbone,
– M est un reste cationique d'origine minérale ou organique, choisi dans le group constitué par l'hydrogène et les cations minéraux dérivés de métaux alcalins ou alcalino-terreux, ou dérivés du plomb, du zinc ou du cuivre, le cation ammonium ($NH_4^+$), les cations ammonium quaternaire,
– $m_1$, $m_2$, et $m_3$ sont des nombres entiers identiques ou différents, pouvant être nuls, inférieurs ou égaux à 5.
– $n_1$, $n_2$, et $n_3$ sont des nombres entiers identiques ou différents, pouvant être nuls, inférieurs ou égaux à 3, l'un au moins des nombres $n_1$ à $n_3$ étant supérieur ou égal à 1.

3. Complexes de formule (I) selon la revendication 2, caractérisés en ce que le coordinat TPPS est au moins une triphénylphosphine sulfonée de formule générale (II) dans laquelle:
– $Y_1$, $Y_2$, et $Y_3$, identiques ou différents représentent des groupement choisis parmi les radicaux alkyles ayant de 1 à 2 atomes de carbone, les radicaux alcoxy ayant de 1 à 2 atomes de carbone et le chlore,
– M représente un cation choisi dans le groupe comprenant le cation hydrogène, le cation ammonium, les cations dérivés du sodium, du potassium, du calcium et du baryum et les cations ammonium quaternaire de formule $N(R_3R_4R_5R_6)$ dans laquelle $R_3$, $R_4$, $R_5$ et $R_6$, identiques ou différents représentent des radicaux alkyles, linéaires ou ramifiés comportant au maximum 4 atomes de carbone,
– $m_1$, $m_2$ et $m_3$ sont des nombres entiers compris entre 0 et 3, inclus.

4. Complexes selon la revendication 2 ou 3, caractérisés en ce que le coordinat TPPS est au moins une triphénylphosphine sulfonée de formule (II) dans laquelle:
– $m_1$, $m_2$ et $m_3$ sont nuls,
– M est choisi parmi les cations dérivant du sodium, du potassium, du calcium, du baryum, le

cation ammonium et les cations tétraméthyl- et tétraéthylammonium;

– le(s) groupement(s) $SO_3$ est (sont) en position méta.

5. Complexes de formule (I) selon l'une quelconque des revendications précédentes caractérisés en ce que les radicaux R et R' sont identiques.

6. Complexes selon l'une quelconque des revendications précédentes caractérisés en ce que les radicaux R et R' sont des radicaux alkyles comportant au maximum 4 atomes de carbone.

7. Complexes de formule (I) selon l'une quelconque des revendications précédentes caractérisés en ce que L est un coordinat carbonyle.

8. Utilisation des complexes rhodiés dinucléaires selon l'une quelconque des revendications précédentes dans les réactions d'hydroformylation de composés organiques comportant une double liaison carbone-carbone.

9. Utilisation d'une solution aqueuse d'au moins un complexe rhodié binucléaire selon l'une quelconque des revendications 1 à 8 dans les réactions d'hydroformylation de composés organiques comportant une double liaison carbone-carbone.

**Revendications pour l'Etats contractant AT**

1. Utilisation de complexes rhodiés dinucléaires et hydrosolubles de formule générale (I) ci-après éventuellement sous forme d'une solution aqueuse dans les réactions d'hydroformylation de composés organiques comportant une double liaison carbone-carbone:

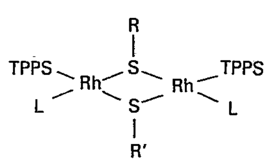

(I)

dans laquelle:

– R et R' identiques ou différents, représentent chacun un radical alkyle, aryle, arylalkyle ou alkylaryle renfermant au maximum 12 atomes de carbone, pouvant comporter un ou plusieurs substituants choisis parmi les atomes d'halogènes, les groupements sulfonate, carboxylate, cyano, amino disubstitué de formule $-NR_1R_2$ dans laquelle $R_1$ et $R_2$, identiques ou différents, représentent chacun un radical alkyle linéaire ou ramifié ayant au maximum 4 atomes de carbone, ammonium et phosphonium, et les radicaux alcoxy qui comportent de 1 à 4 atomes de carbone, R et R' pouvant par ailleurs former ensemble un radical unique divalent alkylène, alcénylène ou alkadiénylène, linéaire ou ramifié contenant de 2 à 6 atomes de carbone,

– TPPS est un coordinat de triphénylphosphine sulfonée et,

– L représente un coordinat carbonyle (CO) ou un coordinat TPPS.

2. Utilisation selon la revendication 1, caractérisée en ce que le coordinat TPPS est au moins une triphénylphosphine sulfonée de formule générale (II):

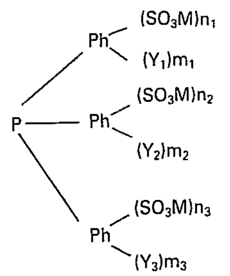

(II)

dans laquelle:

– Ph représente un groupement phényle.

– $Y_1$, $Y_2$, $Y_3$, identiques ou différents, représentent chacun un radical alkyle, linéaire ou ramifié ayant 1 à 4 atomes de carbone, un radical alcoxy comportant de 1 à 4 atomes de carbone, un atome d'halogène, un groupe hydroxyle, un groupe nitrile, un groupe nitro ou un groupe amino disubstitué de formule $-NR_1R_2$ dans laquelle $R_1$ et $R_2$, identiques ou différents, représentent chacun un radical alkyle linéaire ou ramifié ayant au maximum 4 atomes de carbone,

– M est un reste cationique d'origine minérale ou organique, choisi dans le group constitué par l'hydrogène et les cations minéraux dérivés de métaux alcalins ou alcalino-terreux, ou dérivés du plomb, du zinc ou du cuivre, le cation ammonium ($NH_4^+$), les cations ammonium quaternaire,

– $m_1$, $m_2$, et $m_3$ sont des nombres entiers identiques ou différents, pouvant être nuls, inférieurs ou égaux à 5.

– $n_1$, $n_2$, et $n_3$ sont des nombres entiers identiques ou différents, pouvant être nuls, inférieurs ou égaux à 3, l'un au moins des nombres $n_1$ à $n_3$ étant supérieur ou égal à 1.

3. Utilisation selon la revendication 2, caractérisée en ce que le coordinat TPPS est au moins une triphénylphosphine sulfonée de formule générale (II) dans laquelle:

– $Y_1$, $Y_2$, et $Y_3$, identiques ou différents représentent des groupements choisis parmi les radicaux alkyles ayant de 1 à 2 atomes de carbone, les radicaux alcoxy ayant de 1 à 2 atomes de carbone et le chlore,

– M représente un cation choisi dans le groupe comprenant le cation hydrogène, le cation ammonium, les cations dérivés du sodium, du potassium, du calcium et du baryum et les cations ammonium quaternaire de formule $N(R_3R_4R_5R_6)$ dans laquelle $R_3$, $R_4$, $R_5$ et $R_6$, identiques ou différents représentent des radicaux alkyles, linéaires ou ramifiés comportant au maximum 4 atomes de carbones,

– $m_1$, $m_2$ et $m_3$ sont des nombres entiers compris entre 0 et 3, inclus.

4. Utilisation selon la revendication 2 ou 3, caractérisé en ce que le coordinat TPPS est au moins une triphénylphosphine sulfonée de formule (II) dans laquelle:

– $m_1$, $m_2$ et $m_3$ sont nuls,

– M est choisi parmi les cations dérivant du sodium, du potassium, du calcium, du baryum, le

cation ammonium et les cations tétraméthyl- et tétraéthylammonium;
– le(s) groupement(s) $SO_3$ est (sont) en position méta.

5. Utilisation selon l'une quelconque des revendications précédentes caractérisée en ce que dans la formule (I) les radicaux R et R' sont identiques.

6. Utilisation selon l'une quelconque des revendications précédentes caractérisée en ce que dans la formule (I) les radicaux R et R' sont des radicaux alkyles comportant au maximum 4 atomes de carbone.

7. Utilisation selon l'une quelconque des revendications précédentes caractérisée en ce que dans la formule (I) L est un coordinat carbonyle.

**Patentansprüche für die Vertragsstaaten BE, CH, DE, FR, GB, IT, LI, LU, NL, SE**

1. Zweikernige, wasserlösliche Rhodiumkomplexe der allgemeinen Formel (I)

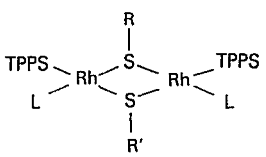
(I)

in der
– R und R' gleich oder verschieden sind und jeweils einen Alkyl-, Aryl-, Arylalkyl- oder Alkylarylrest mit maximal 12 Kohlenstoffatomen bedeuten, der einen oder mehrere Substituenten enthalten kann, ausgewählt aus Halogenatomen, Sulfonat, Carboxylat, Cyano, disubstituierten Aminogruppen der Formel $-NR_1R_2$ in der $R_1$ und $R_2$ gleich oder verschieden sind und jeweils einen linearen oder verzeigten Alkylrest mit maximal 4 Kohlenstoffatomen bedeuten, Ammonium und Phosphonium und Alkylreste mit 1 bis 4 Kohlenstoffatomen, wobei R und R' auch zusammen einen 2-wertigen linearen oder verzweigten Alkylen-, Alkenylen- oder Alkadienylenrest mit 2 bis 6 Kohlenstoffatomen bilden können;
– TPPS einen sulfonierten Triphenylphosphin-Liganden bedeutet und
– L einen Carbonyl(CO)-Liganden oder einen TPPS-Liganden darstellt.

2. Komplexe der Formel (I) nach Anspruch 1, dadurch gekennzeichnet, dass der Ligand TPPS mindestens ein sulfoniertes Triphenylphosphin der allgemeinen Formel (II) ist

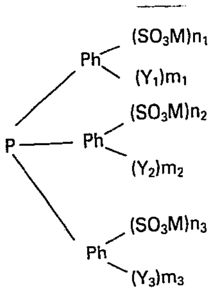
(II)

in der
– Ph eine Phenylgruppe bedeutet
– $Y_1$, $Y_2$ und $Y_3$ gleich oder verschieden sind und jeweils eine lineare oder verzweigte Alkylgruppe mit 1 bis 4 Kohlenstoffatomen, eine Alkoxygruppe mit 1 bis 4 Kohlenstoffatomen, ein Halogenatom, eine Hydroxygruppe, eine Nitrilgruppe, eine Nitrogruppe oder eine disubstituierte Aminogruppe der Formel $-NR_1R_2$ bedeuten, in der $R_1$ und $R_2$ gleich oder verschieden sind und jeweils einen linearen oder verzweigten Alkylrest mit maximal 4 Kohlenstoffatomen darstellen
– M ein kationischer, anorganischer oder organischer Rest ist, ausgewählt aus Wasserstoff, anorganischen Kationen, die abgeleitet sind von Alkali- oder Erdalkalimetallen oder Derivaten von Blei, Zink und Kupfer, dem Ammoniumion ($NH_4^+$) und den quaternären Ammoniumionen
– $m_1$, $m_2$ und $m_3$ gleich oder verschieden sind und ganze Zahlen von 0 bis 5 bedeuten
– $n_1$, $n_2$ und $n_3$ gleich oder verschieden sind und ganze Zahlen von 0 bis 3 bedeuten, wobei mindesten eine der Zahlen $n_1$ bis $n_3$ grösser oder gleich 1 ist.

3. Komplexe der Formel (I) nach Anspruch 2, dadurch gekennzeichnet, dass der Ligand TPPS mindestens ein sulfoniertes Triphenylphosphin der allgemeinen Formel (II) ist, in der
– $Y_1$, $Y_2$ und $Y_3$ gleich oder verschieden sind und Gruppen bedeuten, die ausgewählt sind aus Alkylresten mit 1 bis 2 Kohlenstoffatomen, Alkoxyresten mit 1 bis 2 Kohlenstoffatomen und Chlor;
– M ein Kation bedeutet, ausgewählt aus der Gruppe Wasserstoff, Ammoniumion, Kationen, die abgeleitet sind von Natrium, Kalium, Calcium und Barium und quaternären Ammoniumionen der Formel $N(R_3R_4R_5R_6)$ in der $R_3$, $R_4$, $R_5$ und $R_6$ gleich oder verschieden sind und linear oder verzweigte Alkylreste mit maximal 4 Kohlenstoffatomen bedeuten
– $m_1$, $m_2$ und $m_3$ ganze Zahlen von 0 bis 3 sind.

4. Komplexe nach Anspruch 2 oder 3, dadurch gekennzeichnet, dass der Ligand TPPS mindestens ein sulfoniertes Triphenylphosphin der Formel (II) ist, in der
– $m_1$, $m_2$ und $m_3$ 0 sind
– m ausgewählt ist aus den Kationen, abgeleitet von Natrium, Kalium, Calcium, Barium, dem Ammoniumion und den Tetramethyl- und Tetraethylammoniumionen
– die Gruppe(n) SO sich in m-Stellung befindet(en).

5. Komplexe der Formel (I) nach einem der vorangehenden Ansprüche, dadurch gekennzeichnet, dass in der Formel (I) die Reste R und R' gleich sind.

6. Komplexe nach einem der vorangehenden Ansprüche, dadurch gekennzeichnet, dass in der Formel (I) die Reste R und R' Alkylreste mit maximal 4 Kohlenstoffatomen sind.

7. Komplexe der Formel (I) nach einem der vorangehenden Ansprüche, dadurch gekennzeichnet, dass in der Formel (I) L ein Carbonyl-Ligand ist.

8. Verwendung der zweikernigen Rhodiumkomplexe nach einem der vorangehenden Ansprüche für Hydroformulierungsreaktionen von organischen Verbindungen mit einer Kohlenstoff-Kohlenstoff-Doppelbindung.

9. Verwendung einer wässrigen Lösung mindestens eines zweikernigen Rhodiumkomplexes, nach einem der Ansprüche 1 bis 8 für Hydroformulierungsreaktionen von organischen Verbindungen mit einer Kohlenstoff-Kohlenstoff-Doppelbindung.

**Patentansprüche für den Vertragsstaat AT**

1. Verwendung von zweikernigen, wasserlöslichen Rhodiumkomplexen der allgemeinen Formel (I), gegebenenfalls in Form einer wässrigen Lösung, bei Hydroformylierungsreaktionen von organischen Verbindungen mit einer Kohlenstoff-Kohlenstoff-Doppelbindung:

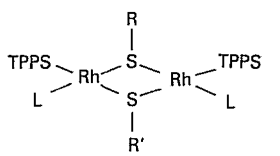
(I)

in der
– R und R′ gleich oder verschieden sind und jeweils einen Alkyl-, Aryl-, Arylalkyl- oder Alkylarylrest mit maximal 12 Kohlenstoffatomen bedeuten, der einen oder mehrere Substituenten enthalten kann, ausgewählt aus Halogenatomen, Sulfonat-, Carboxylat-, Cyano-, disubstituierten Aminogruppen der Formel $-NR_1R_2$ in der $R_1$ und $R_2$ gleich oder verschieden sind und jeweils einen linearen oder verzweigten Alkylrest mit maximal 4 Kohlenstoffatomen bedeuten, Ammonium- und Phosphonium- und Alkylresten mit 1 bis 4 Kohlenstoffatomen, wobei R und R′ auch zusammen einen 2-wertigen linearen oder verzweigten Alkylen-, Alkenylen- oder Alkadienylenrest mit 2 bis 6 Kohlenstoffatomen bilden können;
– TPPS einen sulfonierten Triphenylphosphin-Liganden bedeutet und
– L einen Carbonyl(CO)-Liganden oder einen TPPS-Liganden darstellt.

2. Verwendung nach Anspruch 1, dadurch gekennzeichnet, dass der Ligand TPPS mindestens ein sulfoniertes Triphenylphosphin der allgemeinen Formel (II) ist

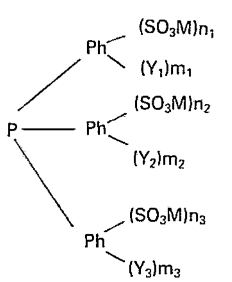
(II)

in der
– Ph eine Phenylgruppe bedeutet,
– $Y_1$, $Y_2$ und $Y_3$ gleich oder verschieden sind und jeweils eine lineare oder verzweigte Alkylgruppe mit 1 bis 4 Kohlenstoffatomen, eine Alkoxygruppe mit 1 bis 4 Kohlenstoffatomen, ein Halogenatom, eine Hydroxygruppe, eine Nitrilgruppe, eine Nitrogruppe oder eine disubstituierte Aminogruppe der Formel $-NR_1R_2$ bedeuten, in der $R_1$ und $R_2$ gleich oder verschieden sind und jeweils einen linearen oder verzweigten Alkylrest mit maximal 4 Kohlenstoffatomen darstellen,
– M ein kationischer, anorganischer oder organischer Rest ist, ausgewählt aus Wasserstoff, anorganischen Kationen, die abgeleitet sind von Alkali- oder Erdalkalimetallen oder Derivaten von Blei, Zink und Kupfer, dem Ammoniumion ($NH_4^+$) und den quaternären Ammoniumionen,
– $m_1$, $m_2$ und $m_3$ gleich oder verschieden sind und ganze Zahlen von 0 bis 5 bedeuten
– $n_1$, $n_2$ und $n_3$ gleich oder verschieden sind und ganze Zahlen von 0 bis 3 bedeuten, wobei mindestens eine der Zahlen $n_1$ bis $n_3$ grösser oder gleich 1 ist.

3. Verwendung nach Anspruch 2, dadurch gekennzeichnet, dass der Ligand TPPS mindestens ein sulfoniertes Triphenylphosphin der allgemeinen Formel (II) ist, in der
– $Y_1$, $Y_2$ und $Y_3$ gleich oder verschieden sind und Gruppen bedeuten, die ausgewählt sind aus Alkylresten mit 1 bis 2 Kohlenstoffatomen, Alkoxyresten mit 1 bis 2 Kohlenstoffatomen und Chlor;
– M ein Kation bedeutet, ausgewählt aus der Gruppe Wasserstoff, Ammoniumion, Kationen, die abgeleitet sind von Natrium, Kalium, Calcium und Barium und quaternären Ammoniumionen der Formel $N(R_3R_4R_5R_6)$ in der $R_3$, $R_4$, $R_5$ und $R_6$ gleich oder verschieden sind und lineare oder verzweigte Alkylreste mit maximal 4 Kohlenstoffatomen bedeuten
– $m_1$, $m_2$ und $m_3$ ganze Zahlen von 0 bis 3 sind.

4. Verwendung nach Anspruch 1 oder 2, dadurch gekennzeichnet, dass der Ligand TPPS mindestens ein sulfoniertes Triphenylphosphin der Formel (II) ist, in der
– $m_1$, $m_2$ und $m_3$ 0 sind
– M ausgewählt ist aus den Kationen, abgeleitet von Natrium, Kalium, Calcium, Barium, dem Ammoniumion und den Tetramethyl- und Tetraethylammoniumionen
– die Gruppe(n) $SO_3$ sich in m-Stellung befinden(en).

5. Verwendung nach einem der vorangehenden Ansprüche, dadurch gekennzeichnet, dass in der Formel (I) die Reste R und R′ gleich sind.

6. Verwendung nach einem der vorangehenden Ansprüche, dadurch gekennzeichnet, dass in der Formel (I) die Reste R und R′ Alkylreste mit maximal 4 Kohlenstoffatomen sind.

7. Verwendung nach einem der vorangehenden Ansprüche, dadurch gekennzeichnet, dass in der Formel (I) L ein Carbonyl-Ligand ist.

**Claims for the Contracting States BE, CH, DE, FR, GB, IT, LI, LU, NL, SE**

1. Dinuclear and water-soluble rhodium complexes of general formula (I):

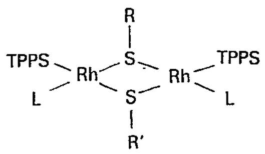 (I)

in which:

– R and R', which are identical or different, each denote an alkyl, aryl, arylalkyl or alkylaryl radical containing at most 12 carbon atoms capable of comprising one or more substituents chosen from among halogen atoms, sulphonate, carboxylate, cyano or disubstituted amino groups of formula $-NR_1R_2$ in which $R_1$ and $R_2$ which are identical or different, each denote a straight-chain or branched alkyl radical containing at most 4 carbon atoms, ammonium and phosphonium and alkoxy radicals which contain from 1 to 4 carbon atoms, R and R' being moreover capable of together forming a single divalent, straight-chain or branched alkylene, alkenylene or alkadienylene radical containing from 2 to 6 carbon atoms,

– TPPS is a sulphonated triphenylphosphine ligand, and

– L denotes a carbonyl (CO) ligand or a TPPS ligand.

2. Complexes of formula (I) according to claim 1, characterized in that the TPPS ligand is at least one sulphonated triphenylphosphine of general formula (II):

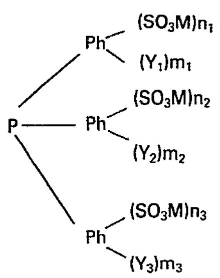 (II)

in which:

– Ph denotes a phenyl group,

– $Y_1$, $Y_2$ and $Y_3$, which are identical or different, each denote a straight-chain or branched alkyl radical containing from 1 to 4 carbon atoms, an alkoxy radical containing from 1 to 4 carbon atoms, a halogen atom, a hydroxyl group, a nitrile group, a nitro group or a disubstituted amino group of formula $-NR_1R_2$ in which $R_1$ and $R_2$, which are identical or different, each denote a straight-chain or branched alkyl radical containing at most 4 carbon atoms,

– M is a cationic residue of inorganic or organic origin, chosen from the group consisting of hydrogen and the inorganic cations derived from alkali metals or alkaline-earth metals, or derived from lead, zinc or copper, the ammonium cation ($NH_4^+$), and quaternary ammonium cations,

– $m_1$, $m_2$ and $m_3$ are identical or different integers capable of being zero, smaller than or equal to 5,

– $n_1$, $n_2$ and $n_3$ are identical or different integers capable of being zero, smaller than or equal to 3, at least one of the numbers $n_1$ to $n_3$ being greater than or equal to 1.

3. Complexes of formula (I) according to claim 2, characterized in that the ligand TPPS is at least one sulphonated triphenylphosphine of general formula (II) in which:

– $Y_1$, $Y_2$ and $Y_3$, which are identical or different, denote groups chosen from alkyl radicals containing from 1 to 2 carbon atoms, alkoxy radicals containing from 1 to 2 carbon atoms, and chlorine,

– M denotes a cation chosen from the group comprising the hydrogen cation, the ammonium cation, the cations derived from sodium, potassium, calcium and barium and the quaternary ammonium cations of formula $N(R_3R_4R_5R_6)$ in which $R_3$, $R_4$, $R_5$ and $R_6$, which are identical or different, denote straight-chain or branched alkyl radicals containing at most 4 carbon atoms, and

– $m_1$, $m_2$ and $m_3$ are integers from 0 to 3 inclusive.

4. Complexes according to claim 2 or 3, characterized in that the ligand TPPS is at least one sulphonated triphenylphosphine of formula (II) in which:

– $m_1$, $m_2$ and $m_3$ are zero,

– M is chosen from cations derived from sodium, potassium, calcium, barium, the ammonium cation and the tetramethyl- and tetraethylammonium cations; and

– the $SO_3$ group(s) is (are) in the meta position.

5. Complexes of formula (I) according to any one of the preceding claims, characterized in that the radicals R and R', are identical.

6. Complexes according to any one of the preceding claims, characterized in that the radicals R and R' are alkyl radicals containing at most 4 carbon atoms.

7. Complexes of formula (I) according to any one of the preceding claims, characterized in that L is a carbonyl ligand.

8. Use of the dinuclear rhodium complexes according to any one of the preceding claims in hydroformylation reactions of organic compounds containing a carbon-carbon double bond.

9. Use of an aqueous solution of at least one dinuclear rhodium complex according to any one of claims 1 to 8 in hydroformylation reactions of organic compounds containing a carbon-carbon double bond.

**Claims for the Contracting States AT**

1. Use of dinuclear and water-soluble rhodium complexes of the following general formula (I) if desired in the form of an aqueous solution in hydroformylation reactions of organic compounds containing a carbon-carbon double bond:

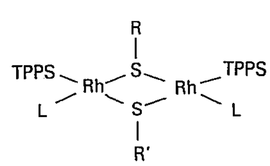 (I)

in which:
– R and R′, which are identical or different, each denote an alkyl, aryl, arylalkyl or alkylaryl radical containing at most 12 carbon atoms capable of comprising one or more substituents chosen from among halogen atoms, sulphonate, carboxylate, cyano or disubstituted amino groups of formula $-NR_1R_2$ in which $R_1$ and $R_2$ which are identical or different, each denote a straight-chain or branched alkyl radical containing at most 4 carbon atoms, ammonium and phosphonium and alkoxy radicals which contain from 1 to 4 carbon atoms, R and R′ being moreover capable of together forming a single divalent, straight-chain or branched alkylene, alkenylene or alkadienylene radical containing from 2 to 6 carbon atoms,
– TPPS is a sulphonated triphenylphosphine ligand, and
– L denotes a carbonyl (CO) ligand or a TPPS ligand.

2. Use according to claim 1, characterised in that the TPPS ligand is at least one sulphonated triphenylphosphine of general formula (II):

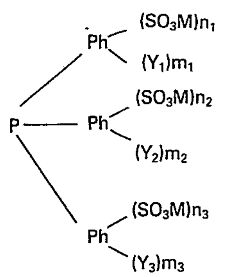

(II)

in which:
– Ph denotes a phenyl group,
– $Y_1$, $Y_2$ and $Y_3$, which are identical or different, each denote a straight-chain or branched alkyl radical containing from 1 to 4 carbon atoms, an alkoxy radical containing from 1 to 4 carbon atoms, a halogen atom, a hydroxyl group, a nitrile group, a nitro group or a disubstituted amino group of formula $-NR_1R_2$ in which $R_1$ and $R_2$, which are identical or different, each denote a straight-chain or branched alkyl radical containing at most 4 carbon atoms,
– M is a cationic residue of inorganic or organic origin, chosen from the group consisting of hydrogen and the inorganic cations derived from alkali metals or alkaline-earth metals, or derived from lead, zinc or copper, the ammonium cation ($NH_4^+$), and quaternary ammonium cations,
– $m_1$, $m_2$ and $m_3$ are identical or different integers capable of being zero, smaller than or equal to 5,
– $n_1$, $n_2$ and $n_3$ are identical or different integers capable of being zero, smaller than or equal to 3, at least one of the numbers $n_1$ to $n_3$ being greater than or equal to 1.

3. Use according to claim 2, characterized in that the ligand TPPS is at least one sulphonated triphenylphosphine of general formula (II) in which:
– $Y_1$, $Y_2$ and $Y_3$, which are identical or different, denote groups chosen from alkyl radicals containing from 1 to 2 carbon atoms, alkoxy radicals containing from 1 to 2 carbon atoms, and chlorine,
– M denotes a cation chosen from the group comprising the hydrogen cation, the ammonium cation, the cations derived from sodium, potassium, calcium and barium and the quaternary ammonium cations of formula $N(R_3R_4R_5R_6)$ in which $R_3$, $R_4$, $R_5$ and $R_6$, which are identical or different, denote straight-chain or branched alkyl radicals containing at most 4 carbon atoms, and
– $m_1$, $m_2$ and $m_3$ are integers from 0 to 3 inclusive.

4. Use according to claim 2 or 3, characterized in that the ligand TPPS is at least one sulphonated triphenylphosphine of formula (II) in which:
– $m_1$, $m_2$ and $m_3$ are zero,
– M is chosen from cations derived from sodium, potassium, calcium, barium, the ammonium cation and the tetramethyl- and tetraethylammonium cations; and
– the $SO_3$ group(s) is (are) in the meta position.

5. Use according to any one of the preceding claims, characterized in that in the formula (I) the radicals R and R′ are identical.

6. Use according to any one of the preceding claims, characterized in that in the formula (I) the radicals R and R′ are alkyl radicals containing at most 4 carbon atoms.

7. Use according to any one of the preceding claims, characterized in that in the formula (I) L is a carbonyl ligand.